# EUROPÄISCHE PATENTANMELDUNG

(11) **EP 1 787 641 A1**
(43) Veröffentlichungstag der Anmeldung: **23.05.2007**
(21) Anmeldenummer: 05025448.1
(22) Anmeldetag: 22.11.2005
(51) Int. Cl.: A61K 9/19

(54) **Tazobactam-Piperacillin-Lyophilisat**

(71) Anmelder: Helm AG, 20097 Hamburg (DE)
(72) Erfinder: Lieb, Sonja, Dr., 20255 Hamburg (DE); Garms, Christian, Dr., 22455 Hamburg (DE); Glänzer, Klaus, Dr., 22337 Hamburg (DE)
(74) Vertreter: Teipel, Stephan

(57) **Zusammenfassung**

Die vorliegende Erfindung betrifft ein Lyophilisat, das Tazobactam und Piperacillin enthält, ein Verfahren zur Herstellung dieses Lyophilisats, die Verwendung dieses Lyophilisats zur Herstellung eines Arzneimittels sowie Arzneimittel, die dieses Lyophilisat umfassen.

## Beschreibung

Die Erfindung betrifft ein Lyophilisat, umfassend Tazobactam und Piperacillin oder deren pharmazeutisch verträgliche Salze, ein Verfahren zur Herstellung dieses Lyophilisats, dessen Verwendung zur Herstellung eines Arzneimittels sowie Arzneimittel, die das Lyophilisat umfassen.

Piperacillin gehört zu den Acylaminopenicillinen, die z.B. in Kliniken zur parenteralen Behandlung von mittleren und schweren Infektionen durch gram-positive oder gramnegative Bakterien eingesetzt werden. Durch übermäßige und falsche Verabreichung von Antibiotika treten in den letzten Jahren immer häufiger Infektionen mit resistenten Bakterienstämmen auf, die durch Einsatz normalerweise verwendeter Penicilline nicht mehr behandelt werden können. Häufigste Ursache der bakteriellen Resistenz, insbesondere gegenüber Acylaminopenicillinen, ist die Bildung von beta-Lactamasen. Diese beta-Lactamasen werden von den resistenten Bakterien ausgeschieden und zerstören die Penicillinwirkstoffe, bevor diese die Bakterien bekämpfen können. Zur Lösung dieses Problems werden unter anderem Kombinationspräparate diverser Penicillinantibiotika mit Hemmstoffen der beta-Lactamasen verabreicht. Hierdurch kann der Resistenzwirkung entgegengewirkt und das Anwendungsspektrum dieser Penicillinantibiotika erweitert werden. Mit Tazobactam steht ein Inhibitor für beta-Lactamasen zur Verfügung, der in Kombination mit bestimmten Penicillinderivaten, insbesondere Piperacillin, ausgezeichnete Wirkung aufzeigt. Tazobactam bindet ebenso wie andere beta-Lactamaseinhibitoren an das aktive Zentrum der beta-Lactamasen. Dabei wird das katalytische Zentrum dieser Enzyme zerstört, und der Hemmstoff wird gespalten. Tazobactam besitzt eine hohe Affinität gegenüber einem breiten Spektrum bakterieller beta-Lactamasen. Insbesondere bindet Tazobactam an beta-Lactamasen aus gram-positiven und gram-negativen sowie aeroben und anaeroben Erregern.

### Tazobactam (a) und Piperacillin (b) weisen die folgenden Strukturformeln auf:

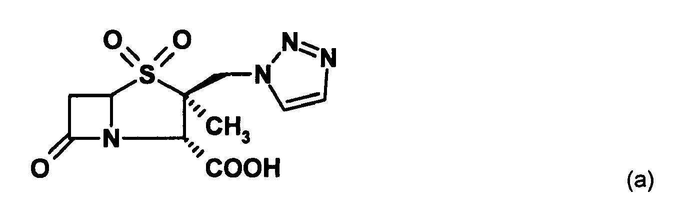

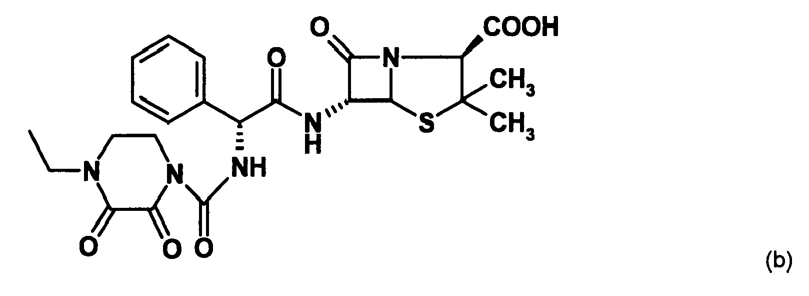

Die Wirkstoffe sind als Mischung unter dem Handelsnamen Zosyn^{®} bzw. Tazobac^{®} zur intravenösen Verabreichung für die Behandlung von moderaten bis schweren Infektionen in Stechfläschchen in einer üblichen Dosis von z.B. 2,5 g oder 4,5 g Tazobactam/Piperacillin-Mischungen, wobei das Mischungsverhältnis 2 g oder 4 g Piperacillin zu 0,5 g Tazobactam beträgt, erhältlich. Aufgrund der Instabilität von Piperacillin in wässriger Lösung bei Raumtemperatur liegen die Wirkstoffe in den Stechfläschchen in fester Form vor. Vor deren Verabreichung müssen die Wirkstoffe daher in einem geeigneten Lösungsmittel gelöst werden.

Tazobactam als Substanz wird in US 4,562,073 unter einer Reihe von Penicillinderivaten offenbart. Diese Druckschrift erwähnt auch die Lyophilisierung von Tazobactam als Monopräparat. Ein Lyophilisierungsprozess für Piperacillin wird in US 4,477,452 und US 4,534,977 offenbart. Diese Dokumente stellen auf Probleme beim Auflösen des Wirkstoffs in einem Stechfläschchen ab und schlagen zur Lösung dieses Problems ein besonders poröses Lyophilisat geringer Dichte vor.

Ein Kombinationspräparat aus Piperacillin und Tazobactam wird in US 6,900,184 offenbart. Dieses Dokument stellt sich die Aufgabe, die Partikelbildung bei der Rekonstitution oder dem Auftauen des Kombinationspräparats zu minimieren. Diese Aufgabe soll durch Zugabe einer Aminocarbonsäure als Chelatisierungsmittel gelöst werden. Das Verfahren zur Herstellung trockener Wirkstoffmischungen wird in diesem US-Patent als nicht wesentlich angesehen, wobei die Lyophilisierung einer Lösung vorgeschlagen wird, die 150-500 mg/ml Piperacillin und 15-125 mg/ml Tazobactam enthalten soll. Von niedrigeren Konzentrationen wird aufgrund angeblicher Nachteile durch steigende Produktionszeiten und damit verbundener Kosten abgeraten. In dem Beispiel dieses US-Patents werden etwa 16,9 ml einer wässrigen Lösung mit etwa 235 mg/ml Piperacillin und etwa 30 mg/ml Tazobactam in Ampullen gefüllt und lyophilisiert.

Ein Lyophilisierungsprozess, der zu einem Lyophilisat einer galactomannanfreien Mischung aus Tazobactam und Piperacillin führt, ist in WO 2005/074925 offenbart.

Ein Problem bei der Herstellung entsprechender pharmazeutischer Präparate liegt in den besonderen physikalisch-chemischen Eigenschaften des Lyophilisats, das nicht fließfähig ist, eine starke Hygroskopizität und eine elektrostatische Aufladung, insbesondere des Lyophilisatfeinstaubs, zeigt. Deshalb kann, wie aus Kostengründen ansonsten üblich, das Lyophilisat nicht als Pulver in Injektionsfläschchen (Vials) gefüllt werden. Vielmehr wird, wie in US 6,900,184 beschrieben, die sterilfiltrierte Lösung der Wirkstoffe selbst in Vials gefüllt und anschließend das Lösungsmittel durch Gefriertrocknung entfernt. Auf diese Weise kann die Pulverabfüllung zwar umgangen werden, allerdings mit dem Nachteil zusätzlicher hoher Kosten bei dem ohnehin teuren Lyophilisierungsprozess. Die Kapazitätsausnutzung der Gefriertrocknung ist unter Einsatz der Viallyophilisierung gegenüber einer Bulklyophilisierung um den Faktor drei schlechter, das heißt die Produktionskosten der Vialgefriertrocknung liegen um das dreifache höher.

Die direkte Vermischung der nicht lyophilisierten, separat gefällten Wirkstoffe Piperacillin-Natrium und Tazobactam-Natrium ist ebenfalls nicht sinnvoll. Neben der sehr hohen, auf die Hygroskopizität der Pulver zurückzuführenden starken Kohäsionstendenz und damit verbundener Inhomogenitäten ist die Auflösegeschwindigkeit der so hergestellten Mischung nämlich viel langsamer als diejenige der im Vial lyophilisierten, die sich normalerweise in weniger als 10 Sekunden im Rahmen der Rekonstitution der Injektion auflöst. Hieraus resultiert das Risiko ungelöster Substanzpartikel in der gebrauchsfertigen Injektionslösung.

Die Vermischung zuvor lyophilisierter Einzelwirkstoffe könnte das Problem der Auflösegeschwindigkeit möglicherweise lösen. Technisch ist diese Möglichkeit wegen der physikalischen Eigenschaften der sehr feinen, amorphen Pulver und der damit verbundenen Probleme beispielsweise von Inhomogenitäten der Mischung wegen mangelnder Fließfähigkeit der Pulver, elektrostatischer Aufladungen sowie Hygroskopizität der Pulver praktisch nicht zugänglich.

Eine Aufgabe der vorliegenden Erfindung ist es deshalb, ein Feststoffgemisch von Piperacillin und Tazobactam zur Verfügung zu stellen, das die Probleme des Standes der Technik nicht aufweist. Das Gemisch soll zur industriellen Weiterverarbeitung, insbesondere zur Trockenabfüllung geeignet sein.

Überraschenderweise wurde gefunden, dass das colyophilisierte Tazobactam/Piperacillin als amorpher, schollenartiger Kuchen anfällt, wenn die Anfangskonzentration der Lösung zu Beginn der Gefriertrocknung < 100 mg/ml, bezogen auf die Gesamtmenge der beiden freien Wirkstoffe, ist. Dieses Bulklyophilisat ergibt nach Siebung beispielsweise über eine kontinuierlich arbeitendende Konus-Siebmaschine (z.B. Glatt Sieb GS, Frewitt TC oder Quadro Comil, s. W.A. Ritschel, A. Bauer-Brandl, "Die Tablette", S. 230ff.; 2. Auflage, Editio Cantor Verlag, Aulendorf) mit einem Siebeinsatz (Lochgröße 0,5 bis 2,5 mm, bevorzugt 0,8 bis 1,2 mm) ein kohäsionsfreies Pulver, das aus kompakten, dichtamorphen Partikeln besteht. Dieses amorphe, sehr gut fließfähige Pulver ist hervorragend für die anschließende Abfüllung in Vials geeignet. Das so erhaltene, zur Einzeldosisabfüllung geeignete Lyophilisat lässt sich durch sein spezifisches Extrusionsvolumen charakterisieren.

Die vorliegende Erfindung betrifft somit ein Lyophilisat, umfassend Tazobactam und Piperacillin oder deren pharmazeutisch verträgliche Salze, das ein spezifisches Extrusionsvolumen von > 200 mm³/g aufweist.

Das erfindungsgemäße Lyophilisat ist kohäsionsfrei, frei fließend zur Einzeldosisabfüllung geeignet sowie bulklagerungs- und transportfähig. Außerdem kommt das Lyophilisat ohne weitere Zusatzstoffe aus.

Figur 1 zeigt die Ergebnisse der Niederdruck-Quecksilberporosimetrie-Untersuchungen für ein Vergleichsbeispiel (Tazobac^{®}) und das erfindungsgemäße Lyophilisat.

Figur 2 zeigt rasterelektronenmikroskopische Aufnahmen von Lyophilisaten bei hundertfacher Vergrößerung, links vom kommerziell erhältlichen Vergleichsbeispiel Tazobac^{®}, das im Vial lyophilisiert wurde, rechts vom erfindungsgemäßen Lyophilisat.

Das spezifische Extrusionsvolumen des erfindungsgemäßen Lyophilisats lässt sich mit der Niederdruck-Quecksilberporosiemetrie bestimmen. Erfindungsgemäß wurde das spezifische Extrusionsvolumen mit einem Pascal 140 (Thermoquest, I) bestimmt. Für die Druckaufbau- und Druckabbauprozedur wurde eine mittlere Geschwindigkeit gewählt (dP= 5; dN 5). Das Porenvolumen der Proben wurde kapazitiv gemessen (Auflösung: 0,1 mm³). Druck und Volumen wurden sowohl bei Druckaufbau als auch bei Druckabbau mit hoher Auflösung aufgezeichnet. Das spezifische Extrusionsvolumen wird aus der Differenz zwischen Endvolumen und totalem kumulativem Volumen sowie der Probeneinwaage berechnet. Es stellt ein Maß für die Pulveraggregation dar. Weitere Einzelheiten der beschriebenen Methode finden sich in "Hg-Niederdruckporosimetrie: Pascal Series Brochure, Thermo Electron Corporation".

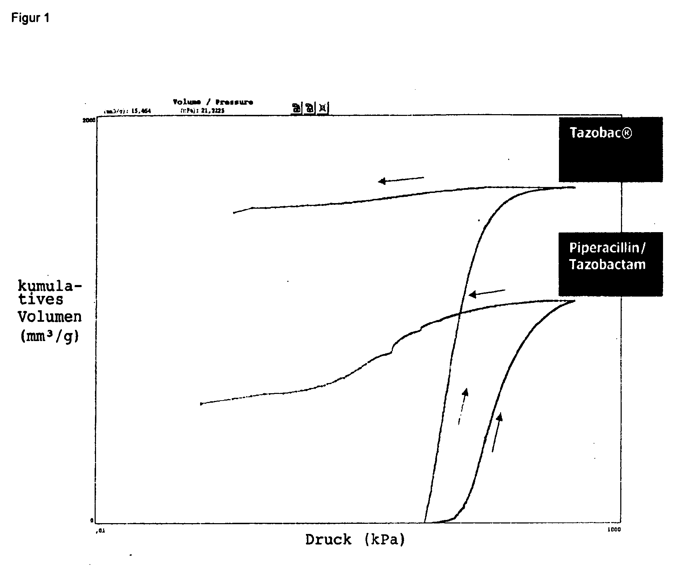

Mittels der vorstehend beschriebenen Methode wurde das spezifische Extrusionsvolumen sowohl eines unter dem Handelsnamen Tazobac^{®} kommerziell erhältlichen Lyophilisats als auch des erfindungsgemäßen Lyophilisats bestimmt. Die Ergebnisse der Messungen sind in Figur 1 wiedergegeben. Die beiden Lyophilisate unterscheiden sich durch die Anfangskonzentration der Lösung zu Beginn der Gefriertrocknung. Man erkennt, dass in die hochporöse Probe des Tazobac^{®} Quecksilber deutlich schneller und auch mit einem höheren Volumen als bei dem erfindungsgemäßen Lyophilisat intrudiert. Bei Tazobac® betrug das kumulative Volumen 1646 mm³/g, wohingegen bei dem erfindungsgemäßen Lyophilisat das kumulative Volumen nur 1089 mm³/g betrug. Das spezifische Extrusionsvolumen für Tazobac^{®} wurde mit 121 mm³/g bestimmt, das spezifische Extrusionsvolumen des erfindungsgemäßen Lyophilisats mit 508 mm³/g.

Erfindungsgemäß weist das Lyophilisat somit ein spezifische Extrusionsvolumen von > 200 mm³/g auf, vorzugsweise von > 300 mm³/g, insbesondere von > 400 mm³/g und besonders bevorzugt von etwa 500 mm³/g.

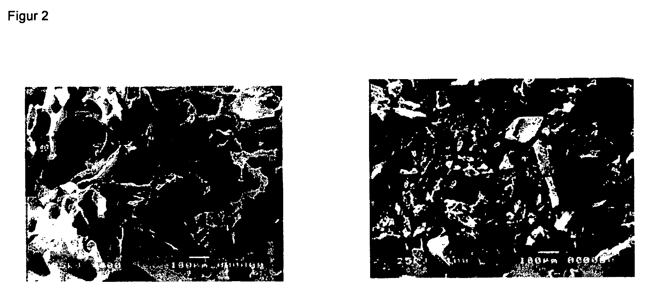

Zur weiteren Charakterisierung des erfindungsgemäßen Lyophilisats wurden SEM-Aufnahmen angefertigt. Die Ergebnisse sind in Figur 2 wiedergegeben. Die nichterfindungsgemäße Tazobac^{®}-Probe (links in Figur 2) zeigt ein für Lyophilisate typisches Aussehen. Überraschenderweise zeigt das erfindungsgemäße Lyophilisat ein völlig anderes Aussehen, wobei das Lyophilisat weniger poröse Partikel mit polygonalen Flächen enthält. Das erfindungsgemäße Lyophilisat ist daher wesentlich besser fließfähig als das herkömmliche Lyophilisat.

Vorzugsweise weist das erfindungsgemäße Lyophilisat darüber hinaus eine wahre Dichte von > 1,38 g/cm³, insbesondere von > 1,40 g/cm³ und besonders bevorzugt von etwa 1,42 g/cm³ auf. Die wahre Dichte kann wie im nachfolgenden Beispiel näher erläutert bestimmt werden. Weitere Einzelheiten können der technischen Broschüre "Pycnometers" der Fa. Quantochrome entnommen werden.

In einer weiteren bevorzugten Ausführungsform weist das erfindungsgemäße Lyophilisat einen Durchgangsparameter D90 von < 70 µm, vorzugsweise im Bereich von 55 - 65 µm auf. Darüber hinaus kann der Durchgangsparameter D10 bei < 8 µm, vorzugsweise im Bereich von 2 - 6 µm und der Durchgangsparameter D50 bei < 35 µm, insbesondere im Bereich von 25 - 35 µm liegen. Die Messung des Durchgangsparameters wird im nachfolgenden Beispiel näher erläutert. Weitere Einzelheiten können der technischen Broschüre "Laser Particle Sizer" der Fa. Fritsch entnommen werden.

Das erfindungsgemäße Lyophilisat sollte sich in einer zur Injektion verabreichbaren üblichen Dosis in einem für Injektionen üblichen Lösungsmittel in weniger als 10 Sekunden auflösen. Damit zeigt es eine zu im Vial lyophilisierte Mischung vergleichbare Auflösungsgeschwindig keit.

Als pharmazeutisch verträgliche Salze der Wirkstoffe eignen sich alle dem Fachmann bekannten Salze. Vorzugsweise wird sowohl das Tazobactam als auch das Piperacillin als Natriumsalz eingesetzt. In einer bevorzugten Ausführungsform enthält das erfindungsgemäße Lyophilisat keinen Aminocarbonsäurechelator oder ein pharmazeutisch verträgliches Salz davon. In einer besonders bevorzugten Ausführungsform enthält das Lyophilisat außer gegebenenfalls einem Puffer keine weiteren Zusatzstoffe, wie beispielsweise Verdünnungsmittel oder Mittel zur Unterstützung der Auflösungsgeschwindigkeit.

Das Gewichtsverhältnis von Tazobactam zu Piperacillin in dem erfindungsgemäßen Lyophilisat kann vom Fachmann in Abhängigkeit vom gewünschten Verwendungszweck gewählt werden. Das Verhältnis kann beispielsweise im Bereich von 1:2 bis 1:10, vorzugsweise bei etwa 1:4 oder etwa 1:8 liegen.

Das erfindungsgemäße Lyophilisat fällt als amorpher, schollenartiger Kuchen an. Dieses Bulklyophilisat ergibt nach Sieben ein kohäsionsfreies Pulver, das aus kompakten, dichtamorphen Partikeln besteht. Anders als herkömmliche Lyophilisate ist dieses Pulver für die anschließende Abfüllung in Vials geeignet. Außerdem ist es bulklagerungs- und transportfähig.

Darüber hinaus hat sich überraschend gezeigt, dass das erfindungsgemäße Lyophilisat weniger hygroskopisch ist als herkömmliche Lyophilisate. Dies wirkt sich zusätzlich positiv auf die Abfülleigenschaften des Produkts aus.

Das erfindungsgemäße Lyophilisat besteht aus einer homogenen Mischung der Wirkstoffe. Im Gegensatz dazu lassen sich getrennt lyophilisierte Wirkstoffpulver nur schwer zu einer homogenen Mischung vereinen, da sie unterschiedliche Partikeldichten aufweisen und es zu elektrostatischen Aufladungen kommt. Das erfindungsgemäße Lyophilisat löst somit die Probleme der Lyophilisate des Standes der Technik.

Das erfindungsgemäße Lyophilisat lässt sich durch Gefriertrocknen einer wässrigen Lösung von Tazobactam und Piperacillin oder deren pharmazeutisch verträglichen Salze herstellen, wobei die Anfangskonzentration der Lösung zu Beginn der Gefriertrocknung < 100 mg/ml, bezogen auf die Gesamtmenge der beiden freien Wirkstoffe, ist. Vorzugsweise ist die Anfangskonzentration < 70 mg/ml, besonders bevorzugt < 50 mg/ml. Die Einzelkonzentrationen der beiden Wirkstoffe können dabei so gewählt werden, dass deren Verhältnis dem gewünschten Verhältnis in der späteren Dosisform entspricht. Dementsprechend kann die Anfangskonzentration an Piperacillin beispielsweise etwa 40 mg/ml oder etwa 20 mg/ml und die Anfangskonzentration an Tazobactam etwa 5 mg/ml betragen. Die bevorzugteste Anfangskonzentration der Lösung zu Beginn der Gefriertrocknung ist dementsprechend im Bereich von 20-50 mg/ml, insbesondere 25-45 mg/ml, bezogen auf die Gesamtmenge der beiden freien Wirkstoffe. Aufgrund der vergleichsweise geringen Konzentration der Wirkstoffe wird das erfindungsgemäße Verfahren vorzugsweise nicht in kleinen Vials oder Ampullen durchgeführt, die gleichzeitig zur Aufbewahrung einer Einheitsdosis der Wirkstoffe dienen, sondern in größeren Behältern, die ein größeres Anfangsvolumen der zu lyophilisierenden Lösung erlauben, wie beispielsweise ein Anfangsvolumen von einigen Litern, z.B. > 10 Liter, vorzugsweise etwa 50 Liter.

Vorzugsweise wird in dem erfindungsgemäßen Verfahren eine Lösung gefriergetrocknet, die einen pH-Wert im Bereich von 5,0 bis 7,0, insbesondere bei etwa 6,0 aufweist. Der pH-Wert der Lösung kann beispielsweise mit Natriumbicarbonat eingestellt werden. Bei der Lösung kann es sich um eine wässrige Lösung handeln. Die sonstigen Verfahrensparameter für die Lyophilisierung können vom Fachmann entsprechend seines Fachwissens frei gewählt werden. Geeignete Verfahrensparameter finden sich beispielsweise in der WO 2005/074925, deren Inhalt daher durch Bezug in die vorliegende Anmeldung aufgenommen wird.

Darüber hinaus betrifft die vorliegende Erfindung ein Lyophilisat, das durch das vorstehend beschriebene Verfahren erhältlich ist, die Verwendung des erfindungsgemäßen Lyophilisats zur Herstellung eines Arzneimittels sowie Arzneimittel, die ein entsprechendes Lyophilisat umfassen. Vorzugsweise wird das erfindungsgemäße Lyophilisat, gegebenenfalls nach weiterer Verarbeitung wie beispielsweise Sieben, dazu in gewünschter Menge von beispielsweise etwa 2,5 g oder etwa 4,5 g bezogen auf die freien Wirkstoffe in Ampullen abgefüllt. Kurz vor Injektion wird die Lösung dann durch Zugabe eines geeigneten Lösungsmittels rekonstituiert.

Die vorliegende Erfindung wird nun durch das nachfolgende Beispiel näher erläutert, wobei dieses nicht als einschränkend zu verstehen ist.

### Beispiel

50 l Wasser qs. wurden in einen rostfreien Stahlreaktor gefüllt und gekühlt. 250 g Tazobactam und 2 kg Piperacillin wurden eingewogen und gut gemischt. Der pH-Wert der Mischung wurde mit Natriumbicarbonat auf 6,0 eingestellt. In steriler Umgebung wurde die Lösung zunächst filtriert, anschließend steril filtriert und lyophilisiert. Der Lyophilisierungskuchen wurde anschließend gemahlen.

Das spezifische Extrusionsvolumen des wie vorstehend beschrieben erhaltenen Lyophilisats wurde mittels eines Pascal 140 (Thermoquest, l) mittels Quecksilber-Niederdruckmessung (0,01 - 350 kPa) bestimmt. Für die Druckaufbau- und Druckabbauprozedur wurde eine mittlere Geschwindigkeit gewählt (dP = 5; dN = 5). Das Porenvolumen der Probe wurde kapazitiv gemessen (Auflösung: 0,1 mm³). Druck und Volumen wurden sowohl bei Druckaufbau als auch bei Druckabbau mit hoher Auflösung aufgezeichnet. Als Vergleichssubstanz wurde das in Vials lyophilisierte und kommerziell erhältliche Tazobac^{®}-Produkt verwendet. Die Ergebnisse der Messung sind in anliegender Figur 1 dargestellt und in nachfolgender Tabelle 1 zusammengefasst.

**Tabelle 1**

| **Probe** | **Spezifisches Extrusionsvolumen [mm³**/g] | **Kumulatives Volumen [mm³**/g] |
|---|---|---|
| Tazobac^{®} | 121 | 1646 |
| erfindungsgemäßes Lyophilisat | 508 | 1089 |

Für die SEM-Untersuchungen wurden die Proben auf ein Kohlenstoff-Paddy aufgebracht und mit Gold besputtert. Die SEM-Aufnahmen wurden im Low-Vacuum-Mode (etwa 8 Pa) aufgenommen. Aufnahmen bei 100-facher Vergrößerung sowohl für Tazobac^{®} als auch für das erfindungsgemäße Lyophilisat sind in Figur 2 wiedergegeben.

Die wahre Dichte der Proben wurde mittels eines Quantachrome Ultrapyknometer 1000 bestimmt. Das Sprüh- und Messgas war Helium. Bei je zwei Parallelanalysen (n = 6) ergab sich für Tazobac^{®} für die wahre Dichte ein Mittelwert von 1,360 g/cm³ und für das erfindungsgemäße Lyophilisat ein Mittelwert von 1,421 g/cm³.

Die Partikelverteilung der Proben wurde mittels einer Fritsch Analysette 22 Compact (Messbereich 0,3 - 300 µm) bestimmt. Die Umwälzpumpe und zugleich Dispergiereinheit war ein Fritsch "Apollo". Die Desagglomerierung erfolgte mit Hilfe eines Ultraschallstabes (Dr. Hilscher GmbH, Ultraschallprozessor UP200H) mit Parameter: 60; 0,5; Zeit: 15 sec.

Es handelt sich hierbei um eine Nassdispergierung im inerten Dispergiermittel Silikonöl (Wacher AK10). Die Probe wurde in einem 50 ml Becherglas unter Zugabe von etwa 25 ml Dispergiermittel mit Hilfe des Ultraschallstabs desagglomeriert und unmittelbar danach der Umwälzpumpe zugeführt. Ab einer Strahlabsorption von 12% startete automatisch die Partikelmessung. Die Hintergrundmessung wurde aktiviert, 7 Scans/Messungen wurden durchgeführt. Die Umwälzpumpe wurde nach jeder Messung gereinigt und mit frischem Silikonöl gefüllt. Es wurden jeweils 3 Analysen durchgeführt, deren gemittelte Werte der Durchgangsparameter in der folgenden Tabelle 2 zusammengefasst sind:

**Tabelle 2**

| **Durchgangsparameter** | **Tazobac^{®}** | **erfindungsgemäßes Lyophilisat** |
|---|---|---|
| D10 [µm] | 10,946 | 4,820 |
| D50 [µm] | 41,559 | 29,874 |
| D90 [µm] | 82,712 | 61,781 |

Schließlich wurde mittels Karl-Fischer-Titration der Wassergehalt der Proben bestimmt. Für die Analyse wurde ein Mettler Toledo DL38 Titrator verwendet. Zu Beginn wurde eine Konzentrationsbestimmung mit Oxalsäure durchgeführt und anschließend die Proben analysiert (n = 3). Die Ergebnisse sind in nachfolgender Tabelle 3 zusammengefasst.

**Tabelle 3**

| | **Tazobac^{®}** | | **erfindungsgemäßes Lyophilisat** | |
|---|---|---|---|---|
| **Konditionierung** | As is | 43% RF | As is | 43%RF |
| 1. Analyse | 1,88% | 4,69% | 1,68% | 4,11% |
| 2. Analyse | 1,73% | 4,65% | 1,85% | 4,12% |
| 3. Analyse | 1,70% | 4,53% | 1,56% | 4,15% |
| **Mittelwert** | **1,77%** | **4,63%** | **1,70%** | **4,13%** |

Man erkennt, dass beide Proben zunächst einen ähnlichen Wassergehalt aufwiesen. Ohne Vorkonditionierung, d.h. die Proben wurden direkt aus einem vorher geschlossenen Behälter entnommen, liegen die Werte bei etwa 1,7% (Wasser bezogen auf feuchte Ausgangssubstanz). Nach der Konditionierung bei 43% relativer Feuchtigkeit (RF) über 15 Stunden stieg der Wassergehalt in Tazobac^{®} auf 4,63%, wohingegen der Wassergehalt in dem erfindungsgemäßen Lyophilisat auf nur 4,13% stieg. Das erfindungsgemäße Lyophilisat ist daher weniger hygroskopisch als das herkömmliche Lyophilisat.

## Patentansprüche

1. Lyophilisat, umfassend Tazobactam und Piperacillin oder deren pharmazeutisch verträgliche Salze, **dadurch gekennzeichnet, dass** das Lyophilisat ein spezifisches Extrusionsvolumen von > 200 mm³/g aufweist.

2. Lyophilisat nach Anspruch 1, **dadurch gekennzeichnet, dass** es eine wahre Dichte von > 1,38 g/cm³ aufweist.

3. Lyophilisat nach Anspruch 1 oder 2, **dadurch gekennzeichnet, dass** es einen Durchgangsparameter D90 von < 70 µm aufweist.

4. Lyophilisat nach einem der vorhergehenden Ansprüche, **dadurch gekennzeichnet, dass** es partikelförmig und fließfähig ist.

5. Lyophilisat nach einem der vorhergehenden Ansprüche, **dadurch gekennzeichnet, dass** es sich in einer zur Injektion verabreichbaren üblichen Dosis in weniger als 10 Sekunden auflöst.

6. Lyophilisat nach einem der vorhergehenden Ansprüche, **dadurch gekennzeichnet, dass** das Tazobactam und das Piperacillin als deren Natriumsalze vorliegen.

7. Lyophilisat nach einem der vorhergehenden Ansprüche, **dadurch gekennzeichnet, dass** es keinen Aminocarbonsäurechelator oder ein pharmazeutisch verträgliches Salz davon umfasst.

8. Lyophilisat nach einem der vorhergehenden Ansprüche, **dadurch gekennzeichnet, dass** das Gewichtsverhältnis von Tazobactam zu Piperacillin im Bereich von 1:6 bis 1:10, vorzugsweise bei etwa 1:4 oder etwa 1:8 liegt.

9. Verfahren zur Herstellung eines Lyophilisats wie in einem der vorhergehenden Ansprüche definiert, umfassend das Gefriertrocknen einer wässrigen Lösung von Tazobactam und Piperacillin oder deren pharmazeutisch verträglichen Salze, **dadurch gekennzeichnet, dass** die Anfangskonzentration der Lösung zu Beginn der Gefriertrocknung < 100 mg/ml, bezogen auf die Gesamtmenge der beiden freien Wirkstoffe, ist.

10. Verfahren nach Anspruch 9, **dadurch gekennzeichnet, dass** der pH-Wert der Lösung im Bereich von 5,0 bis 7,0, vorzugsweise bei etwa 6,0 liegt.

11. Lyophilisat, erhältlich durch das Verfahren nach einem der Ansprüche 9 oder 10.

12. Verwendung eines Lyophilisats nach einem der Ansprüche 1-8 oder 11 zur Herstellung eines Arzneimittels.

13. Arzneimittel, umfassend ein Lyophilisat nach einem der Ansprüche 1-8 oder 11.
